# EUROPÄISCHE PATENTSCHRIFT

(11) **EP 0 607 748 B1**
(45) Veröffentlichungstag und Bekanntmachung des Hinweises auf die Patenterteilung: **23.06.1999**
(21) Anmeldenummer: 93810039.3
(22) Anmeldetag: 21.01.1993
(51) Int. Cl.: A61F 2/42

(54) **Künstliches Handgelenk**
Artificial wrist joint
Prothèse de poignet

(43) Veröffentlichungstag der Anmeldung: 27.07.1994
(73) Patentinhaber: Sulzer Orthopädie AG, 6340 Baar (CH)
(72) Erfinder: Sennwald, Gontran, Dr.med., CH-9000 St. Gallen (CH); Horber, Willi, CH-8005 Zürich (CH); Koch, Rudolf, CH-6343 Buonas (CH)
(74) Vertreter: Heinen, Detlef

(56) Entgegenhaltungen:
- WO-A-90/11062
- FR-A- 2 661 817
- GB-A- 2 166 654
- US-A- 3 916 451
- US-A- 4 040 130
- US-A- 4 085 466
- US-A- 4 106 128
- US-A- 4 224 696
- US-A- 4 259 752

## Beschreibung

Die Erfindung handelt von einem künstlichen Handgelenk zur Erzeugung einer Flexions- und Extensionsbewegung mit einem distalen sphärischen Lagerkörper, mit einer proximalen an der Speiche befestigten Führungsfläche und mit einem Zwischenkörper, der gegen distal zum Lagerkörper eine sphärische Gegenfläche und gegen proximal eine Gleitfläche aufweist.

Ein künstliches Handgelenk ist in der Patentschrift US 4,040,130 in Form eines beschränkten Kugelgelenks gezeigt. Eine Gelenkkugel ist dort über ein Joch und Knochennägel mit mehreren Metakarpal Knochen verbunden. Die Bewegung der Gelenkkugel ist gegenüber einer Zwischenschale, und die der Zwischenschale gegenüber der im Radius verankerten Lagerschale durch kreuzförmig zueinander angeordneten Nuten, in denen Führungsknochen nicht verdrehbar gleiten, beschränkt. Das Gelenk erlaubt die Schwenkung zum zwei starre Achsen, die sich im Kugelgelenk kreuzen. Das Einsetzen eines derartigen Implantats stellt für ein Handgelenk einen grossen Eingriff dar, der nicht bei allen Erkrankungen des Handgelenks gerechtfertigt ist. Gerade bei arthritischen Erkrankungen des Handgelenks, bei denen eine Verringerung der Schmerzen gegenüber einer beschränkten Funktion des Handgelenks im Vordergrund steht, wäre ein reduzierter Eingriff von Vorteil.

Eine weitere Ausführung eines künstlichen Handgelenks ist in der FR-A-2 661 817 gezeigt. Ein doppeltes Kugelgelenk mit einer Kugel und zwei konzentrischen Kugelschalen ist durch zusätzliche Parallelführungen an den Lagerflächen so eingeschränkt, dass zwei zueinander annähernd senkrecht stehende Scharniergelenke entstehen, deren Achsen sich im Kugelmittelpunkt kreuzen. Die Parallelführungen weisen Endanschläge auf, die die Schwenkbewegung des Gelenks um jede Drehachse jeweils begrenzen. Der Nachteil dieses Gelenks ist darin zu sehen, dass senkrecht zu der Ebene von Elle und Speiche im Gegensatz zum natürlichen Handgelenk eine ortsfeste Drehachse besteht, die zu einer anderen Belastung der Bänder führt. Die Erfindung hat daher die Aufgabe, mit wenig Verlust an Knochenmaterial ein Implantat zu schaffen, bei dem die wesentlichen Bewegungen des Handgelenks erhalten bleiben. Diese Aufgabe wird mit den Kennzeichen des unabhängigen Anspruchs 1 gelöst, wobei die im Oberbegriff definierten Merkmale aus der FR-A-2 661 817 bekannt sind.

Die Erfindung hat den Vorteil, dass es sich um einen kleineren Eingriff handelt, indem hauptsächlich in der proximalen Karpalreihe Scaphoid, Lunatum und Triquetrum entfernt werden, während der Bandapparat weitgehend erhalten bleibt und nach einer natürlichen Verkürzung alle wesentlichen Funktionen weiterhin erfüllen kann. Ausserdem entsteht eine geringere Versteifung des Handgelenks, indem die natürlichen Bewegungsmöglichkeiten zwischen den verbleibenden Handgelenkknochen und den Metakarpals erhalten bleiben, während die Führung des implantierten Gelenkanteils analog zum natürlichen Gelenk von der Streckung von Sehnen und Bändern abhängig ist.

Der künstliche Gelenkanteil besteht aus einem distalen sphärischen Lagerkörper, welcher mit Befestigungsmitteln am Kopfbein oder/und Hakenbein verankerbar ist, sowie aus einer Plattform, welche an der Speiche befestigt ist und quer zur Längsachse der Speiche in Richtung zur Elle eine Führungsfläche aufweist. Ein Zwischenkörper stützt sich proximal mit einer Gleitfläche auf der Führungsfläche ab und ist in Richtung zur Elle verschiebbar, während er distal ein sphärisches Lager für den Lagerkörper bildet. Da nur ein geringer Anteil der Handgelenkknochen entfernt wird, bleibt der Bandapparat erhalten und ermöglicht eine offene Führung.

Weitere vorteilhafte Weiterbildungen sind mit den Kennzeichen der Unteransprüche 2 bis 8 aufgeführt.

Im folgenden wird die Erfindung anhand von Ausführungsbeispielen erklärt. Es zeigen:
- Fig. 1: schematisch eine Draufsicht auf den vergrösserten Ausschnitt eines Handgelenks in der von Speiche und Elle gebildeten Ebene;
- Fig. 2: schematisch in einer Seitenansicht die Prothesenteile der Anordnung nach Figur 1;
- Fig. 3: schematisch eine Draufsicht analog zu Figur 1 mit einem zu seiner Längsachse rotationssymmetrischen Zwischenkörper, und
- Fig. 4: schematisch in einer Seitenansicht die Prothesenteile der Anordnung nach Figur 3.

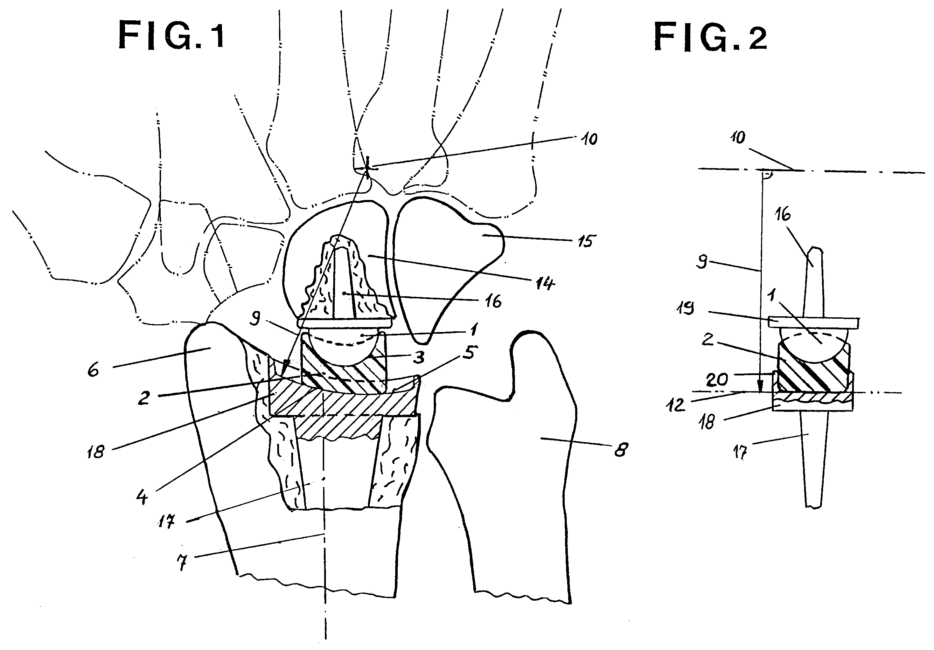

Die Figuren 1 und 2 zeigen ein Handgelenk, bei dem in der proximalen Karpalreihe Scaphoid, Lunatum und Triquetrum durch einen Zwischenkörper 2 ersetzt sind, der sich gegen proximal auf einer Plattform 18 mit Führungsfläche 5 abstützt, welche an der Speiche 6 mit einer Verankerung 17 verankert ist. Die Führungsfläche 5 und Seitenbänder 20, welche die Plattform 18 begrenzen, erlauben dem Zwischenkörper 2 auf seiner Gleitfläche 4 eine Verschiebung quer zur Längsachse 7 der Speiche 6 in Richtung zur Elle 8. Gleitfläche 4 und Führungsfläche 5 besitzen eine gemeinsame Erzeugende 12, die um eine Achse 10, welche senkrecht zur gemeinsamen Ebene von Speiche und Elle steht, drehbar ist. Die Erzeugende 12 ist in diesem Fall ein Geradenabschnitt der mit Radius 9 um die Achse 10 schwenkbar ist. Dabei ist der Radius 9 so gross bemessen, dass die Achse 10 ausserhalb des eigentlichen Gelenks gegen distal angeordnet ist.

Am Kopfbein 14 ist ein sphärischer Lagerkörper 1 mit einem Stift 16 an einer Resektionsfläche verankert. Der Lagerkörper 1 hat gegen proximal einen Kugelausschnitt, der durch einen vorstehenden Kragen 19 abgeschlossen wird.

Eine Gegenfläche 3 im Zwischenkörper 2 bildet eine Lagerschale, deren Rand in Richtung der Flexion/Extension einen mittleren Abstand zu Kragen 19 aufweist, welcher ein Mehrfaches des mittleren Abstandes in Richtung Abduktion/Adduktion beträgt. Das geringe Spiel zwischen Rand und Kragen 19 in Richtung Abduktion/Adduktion bewirkt, dass eine Schwenkbewegung beim Anschlagen des Kragens zwischen Gleitfläche 4 und Führungsfläche 5 fortgesetzt wird, während bei einer Flexions- und Extensionsbewegung die Schwenkung durch den grösseren Abstand zwischen Rand und Kragen 19 ermöglicht wird.

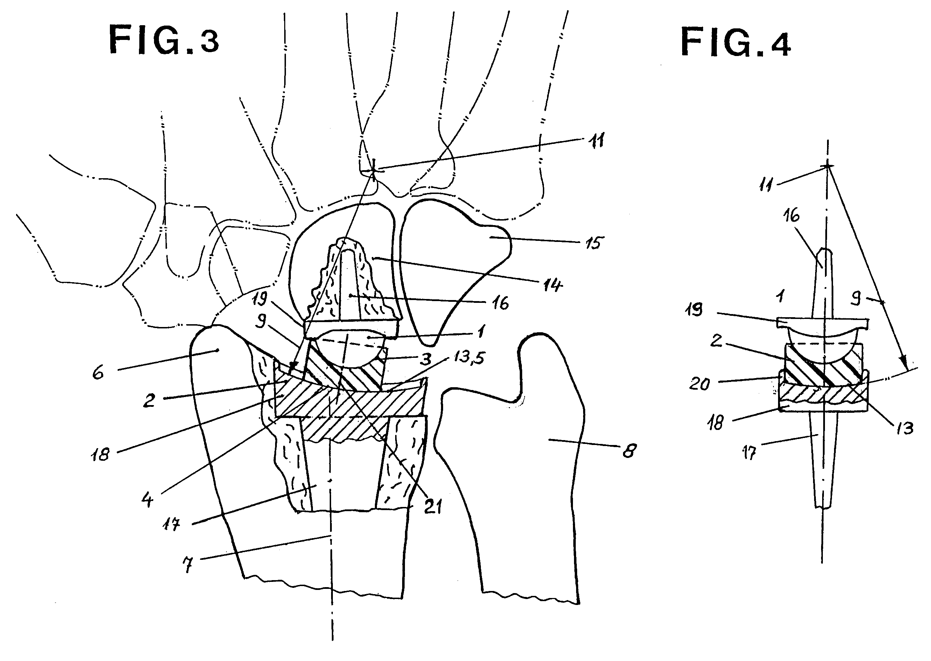

In den Figuren 3 und 4 sind in der proximalen Karpalreihe Scaphoid, Lunatum und Triquetrum durch einen Zwischenkörper 2 ersetzt, der sich gegen proximal mit einer Gleitfläche 4 auf einer Plattform 18 abstützt, welche mit der Gleitfläche 4 eine gemeinsame sphärische Fläche 13 als Führungsfläche 5 besitzt, deren Mittelpunkt 11 mit Radius 9 aus dem Bereich des Gelenks heraus gegen distal angeordnet ist. Die sphärische Fläche 13 ist durch Seitenbänder 20 begrenzt und erlaubt eine Verschiebung des Zwischenkörpers 2 quer zur Längsachse 7 der Speiche 6 in Richtung zur Elle 8. Der Zwischenkörper 2 ist in Bezug auf seine Längsachse 21 rotationssymmetrisch ausgeführt und bildet eine sphärische Gegenfläche 3 zu einem sphärischen Lagerkörper 1, welcher einen vorstehenden Kragen 19 aufweist und mit einem Stift 16 am Hauptbein 14 verankert ist. Der Kragen 19 ist in Richtung der Abduktion/Adduktion weiter gegen den Zwischenkörper 2 heruntergezogen, sodass als Wegbegrenzung der mittlere Abstand zwischen Kragen 19 und Zwischenkörper 2 in Richtung Flexion/Extension ein Mehrfaches des mittleren Abstandes in Richtung Abduktion/Adduktion beträgt. In Figur 3 ist der Kragen 19 soweit zur Daumenseite geschwenkt, dass er auf dem Rand des Zwischenkörpers 2 aufsitzt.

Dadurch dass Kopfbein 14 und Hakenbein 15 gegenüber den mit durchbrochenen Linien angedeuteten Metakarpals unverändert sind, bleibt der Bandapparat weitgehend erhalten und entsteht eine Beweglichkeit die dem natürlichen Gelenk nahe kommt, während die direkten Reibstellen an dem Knochen von Speiche und Elle auf die Prothese verlagert worden sind. Soweit es der Bandapparat erlaubt, ist auch eine begrenzte Schwenkung des Lagerkörpers 1 um seine Längsachse möglich.

## Patentansprüche

1. Künstliches Handgelenk zur Erzeugung einer Flexions- und einer Extensionsbewegung mit einem distalen sphärischen Lagerkörper (1), mit einer proximalen an einer Speiche (6) befestigbaren Führungsfläche (5) und mit einem Zwischenkörper (2), der gegen distal zum Lagerkörper (1) eine sphärische Gegenfläche (3) und gegen proximal eine Gleitfläche (4) aufweist, wobei der sphärische Lagerkörper (1) am Kopfbein (14) oder/und Hakenbein (15) verankerbar ist, der Zwischenkörper (2) in der proximalen Karpalreihe Scaphoid, Lunatum und Triquetrum ersetzt und die Gleitfläche (4) quer zur Längsachse (7) der Speiche (6) in Richtung zur Elle (8) in der Führungsfläche (5) geführt verschiebbar ist, dadurch gekennzeichnet, dass der sphärische Lagerkörper (1) einen Kragen (19) aufweist, der als Wegbegrenzung die Schwenkbewegungen zwischen Lagerkörper (1) und Zwischenkörper (2) einschränkt und dass Führungsfläche (5) und Gleitfläche (4) durch eine gemeinsame Erzeugende (12) gebildet sind, welche um eine Achse (10), die senkrecht zur gemeinsamen Ebene von Speiche (6) und Elle (8) steht, drehbar ist, wobei die Achse (10) ausserhalb des künstlichen Handgelenks gegen distal angeordnet ist.

2. Künstliches Handgelenk nach Anspruch 1, dadurch gekennzeichnet, dass die Führungsfläche (5) durch Seitenbänder (20) ergänzt ist.

3. Künstliches Handgelenk nach Anspruch 1 oder 2, dadurch gekennzeichnet, dass der mittlere Abstand zwischen Kragen (19) und Zwischenkörper (2) in Richtung der Flexion/Extension ein Mehrfaches des mittleren Abstandes in Richtung Abduktion/Adduktion beträgt.

4. Künstliches Handgelenk nach einem der Ansprüche 1 bis 3, dadurch gekennzeichnet, dass Führungsfläche (5) und Gleitfläche (4) auf Ausschnitten einer gemeinsamen sphärischen Fläche (13) liegen, deren Mittelpunkt (11) in einer gemeinsamen Ebene von Speiche (6) und Elle (8) ausserhalb des künstlichen Handgelenks gegen distal angeordnet ist.

5. Künstliches Handgelenk nach Anspruch 4, dadurch gekennzeichnet, dass der Zwischenkörper (2) bezüglich seiner Längsachse (21) rotationssymmetrisch ausgeführt ist.

6. Künstliches Handgelenk nach einem der Ansprüche 1 bis 4, dadurch gekennzeichnet, dass der sphärische Lagerkörper (1) relativ zum Zwischenkörper (2) um dessen Längsachse (21) begrenzt schwenkbar ist.

## Claims

1. An artificial wrist joint for producing a flexion and an extension movement having a distal spherical bearing member (1), having a proximal guide surface (5) attachable to a radius (6) and having an intermediate member (2), which at the distal end towards the bearing member (1) comprises a spherical counter-surface (3) and at the proximal end comprises a sliding surface (4), wherein the spherical bearing member (1) can be attached to the capitate bone (14) or/and hamate bone (15), the intermediate member (2) replaces the scaphoid, lunate and triquetral bones in the proximal carpal row and the sliding surface (4) can be displaced at right angles to the longitudinal axis (7) of the radius (6) towards the ulna (8) by being guided in the guide surface (5).
**characterised in that** the spherical bearing member (1) comprises a collar (19), which as a displacement stop restricts the swivel movements between bearing member (1) and intermediate member (2),
**and in that** guide surface (5) and sliding surface (4) are formed by a common generatrix (12), which is rotatable about an axis (10) which is perpendicular to the common plane of radius (6) and ulna (8), with the axis (10) being disposed outside the artificial wrist joint towards the distal end.

2. An artificial wrist joint according to Claim 1,
**characterised in that** the guide surface (5) is supplemented by collateral ligaments (20).

3. An artificial wrist joint according to Claim 1 or 2,
**characterised in that** the mean distance between collar (19) and intermediate member (2) in the flexion/extension direction is a multiple of the mean distance in the abduction/adduction direction.

4. An artificial wrist joint according to one of Claims 1 to 3,
**characterised in that** guide surface (5) and sliding surface (4) lie on cut-outs of a common spherical surface (13), the centre point (11) of which is disposed in a plane common to radius (6) and ulna (8) outside the artificial wrist joint towards the distal end.

5. An artificial wrist joint according to Claim 4,
**characterised in that** the intermediate member (2) is designed rotationally symmetrical with respect to its longitudinal axis (21).

6. An artificial wrist joint according to one of Claims 1 to 4,
**characterised in that** the spherical bearing member (1) can be swivelled to a limited extent relative to the intermediate member (2) about its longitudinal axis (21).

## Revendications

1. Poignet artificiel pour produire un mouvement de flexion et d'extension avec un corps de palier sphérique distal (1), une surface de guidage proximale (5) pouvant être fixée à un radius (6) et avec un corps intermédiaire (2) qui présente une contre-surface sphérique (3) s'étendant distalement vers le corps de palier (1) et une surface de glissement (4) s'étendant proximalement, où le corps de palier sphérique (1) peut être ancré au grand os du carpe (14) et/ou à l'os crochu (15), où le corps intermédiaire (2) dans la rangée carpienne proximale remplace le scaphoïde, le lunatum et le triquetrum, et la surface de glissement (4) est déplacable d'une manière guidée transversalement à l'axe longitudinal (7) du radius (6) en direction vers le cubitus (8) dans la surface de guidage (5), caractérisé en ce que le corps de palier sphérique (1) présente un col (19) qui, en tant que délimitation de trajet, limite les mouvements de pivotement entre le corps de palier (1) et le corps intermédiaire (2), et en ce que la surface de guidage (5) et la surface de glissement (4) sont formées par une génératrice commune (12) qui peut tourner autour d'un axe (10) qui s'étend perpendiculairement au plan commun du radius (6) et du cubitus (8), où l'axe (10) est disposé dans le sens distal à l'extérieur du poignet artificiel.

2. Poignet artificiel selon la revendication 1, caractérisé en ce que la surface de guidage (5) est complétée par des bandes latérales (20).

3. Poignet artificiel selon la revendication 1 ou 2, caractérisé en ce que l'écart moyen entre le col (19) et le corps intermédiaire (2), dans la direction de la flexion/extension représente un multiple de l'écart moyen dans la direction abduction/adduction.

4. Poignet artificiel selon l'une des revendications 1 à 3, caractérisé en ce que la surface de guidage (5) et la surface de glissement (4) se situent sur des découpures d'une surface sphérique commune (13) dont le centre (11) est disposé distalement dans un plan commun formé par le radius (6) et le cubitus (8) à l'extérieur du poignet artificiel.

5. Poignet artificiel selon la revendication 4, caractérisé en ce que le corps intermédiaire (2), relativement à son axe longitudinal (21), est réalisé avec une symétrie en rotation.

6. Poignet artificiel selon l'une des revendications 1 à 4, caractérisé en ce que le corps de palier sphérique (1), relativement au corps intermédiaire (2), peut pivoter d'une manière limitée autour de l'axe longitudinal (21) de celui-ci.
